# EUROPEAN PATENT APPLICATION

(11) **EP 4 393 525 A1**
(43) Date of publication of application: **03.07.2024**
(21) Application number: 22861633.0
(22) Date of filing: 18.08.2022
(51) Int. Cl.: A61M 1/00, A61M 1/02, B01L 3/00

(54) **PISTON FOR CENTRIFUGATION AND CENTRIFUGATION DEVICE INCLUDING SAME**

(30) Priority: 26.08.2021 KR 20210112893
(71) Applicant: Bslrest Inc., Gimhae-si, Gyeongsangnam-do 50969 (KR)
(72) Inventor: LEE, Jun Seok, Busan 48504 (KR)
(74) Representative: Biallo, Dario
(86) International application number: PCT/KR2022/012343
(87) International publication number: WO 2023/027423

(57) **Abstract**

A piston for centrifugation according to an embodiment may comprise: a piston body including an inner body surface; a valve including an outer valve surface and a recess part; and an inner sealing part which is positioned at the recess part, is in contact with the inner body surface, is configured to space the outer valve surface apart from the inner body surface, and is configured to, at the time of centrifugation, seal a gap between the inner body surface and the outer valve surface.

## Description

### Technical Field

The present disclosure relates to a piston for centrifugation and a centrifugation device including the piston.

### Background Art

Since fatty tissue obtained from a living body by aspiration and incision contains a mixture of impurities (e.g., oil), technology to remove impurities from the fatty tissue obtained from the living body is being developed to obtain pure fatty lump. For example, Korean Patent Application Publication No. 10-2015-0122102 discloses a piston of a syringe for liposuction implantation.

### Disclosure of the Invention

### Technical Problem

One aspect of the present disclosure may provide a piston for centrifugation and a centrifugation device including the piston to remove impurities from fatty tissue obtained from a living body and obtain pure fatty lump.

### Solution to Problem

A piston for centrifugation according to an embodiment includes a piston body having an outer body surface and an inner body surface, in which the piston body includes a hollow part defined by the inner body surface, in which the piston body includes a first opening positioned at the outer body surface, a second opening defined by at least a portion of the outer body surface and connected to the inner body surface and the hollow part, and a first passage connecting the first opening to the hollow part, a valve configured to open or block the first passage and positioned at the hollow part to be spaced apart from the inner body surface, in which the valve has an outer valve surface, in which the outer valve surface defines the first passage and a second passage, in which the first passage is positioned between the inner body surface and the outer valve surface, in which the second passage is fluidly connected to the second opening, in which the valve includes a recess part formed in the outer valve surface, and a first inner sealing part positioned at the recess part, in contact with the inner body surface, configured to space the outer valve surface apart from the inner body surface, and configured to, at a time of centrifugation, seal a gap between the inner body surface and the outer valve surface.

In an embodiment, the piston may further include a first fixing part positioned on the inner body surface and configured to restrict movement of the valve toward the second opening.

In an embodiment, the valve may include a first valve body part including the recess part and a second valve body part connected to the first valve body part, and the first fixing part may include a base part positioned on the inner body surface and a protruding part protruding from the base part and configured to contact the first valve body part.

In an embodiment, the first valve body part may contact the protruding part so that a size of the gap between the inner body surface and the outer valve surface is from 5 micrometers (µm) to 150 µm.

In an embodiment, the inner body surface may include a first inner body surface and a second inner body surface that is recessed from the first inner body surface and forms a first step with the first inner body surface, and the first fixing part may be positioned on the second inner body surface.

In an embodiment, the piston may further include a second inner sealing part positioned on the inner body surface and including a first part and a second part that is opposite to the first part and a first fixing part positioned on the inner body surface, configured to support a first part of the second inner sealing part, and configured to fix the second inner sealing part to the inner body surface.

In an embodiment, the piston may further include a second fixing part positioned on the inner body surface, configured to support a second part of the second inner sealing part, and configured to fix the second inner sealing part to the inner body surface.

In an embodiment, the piston body may include a first engaging part, and the second fixing part may include a second engaging part configured to engage with the first engaging part.

In an embodiment, the second fixing part may include a fastening part.

A centrifugation device with a rotation shaft according to an embodiment includes a syringe configured to rotate with respect to the rotation shaft, in which the syringe includes a container capable of holding a biological tissue and a piston for centrifugation movably positioned inside the container, in which the piston includes a piston body having an outer body surface and an inner body surface, in which the piston body includes a hollow part defined by the inner body surface, in which the piston body includes a first opening positioned at the outer body surface, a second opening defined by at least a portion of the outer body surface and connected to the inner body surface and the hollow part, and a first passage connecting the first opening to the hollow part, a valve configured to open or block the first passage and positioned at the hollow part to be spaced apart from the inner body surface, in which the valve has an outer valve surface, in which the outer valve surface defines the first passage and a second passage, in which the first passage is positioned between the inner body surface and the outer valve surface, in which the second passage is fluidly connected to the second opening, in which the valve includes a recess part formed in the outer valve surface, and a first inner sealing part positioned at the recess part, in contact with the inner body surface, configured to space the outer valve surface apart from the inner body surface, and configured to, at a time of centrifugation, seal a gap between the inner body surface and the outer valve surface.

In an embodiment, the piston may further include a second inner sealing part positioned on the inner body surface and including a first part and a second part that is opposite to the first part, a first fixing part positioned on the inner body surface, configured to support a first part of the second inner sealing part, and configured to fix the second inner sealing part to the inner body surface, a second fixing part positioned on the inner body surface, configured to support a second part of the second inner sealing part, and configured to fix the second inner sealing part to the inner body surface, and a plunger configured to be fastened to the second fixing part, in which the second inner sealing part may contact the plunger and be configured to seal a gap between the inner body surface and the plunger.

### Advantageous Effects of Invention

A piston for centrifugation and a centrifugation device including the piston according to an embodiment may provide improved sealing between a front space and a rear space of the piston while centrifugation is performed on fatty tissue obtained from a living body. The effects of the piston for centrifugation and the centrifugation device including the piston according to an embodiment may not be limited to the above-mentioned effects, and other unmentioned effects may be clearly understood from the following description by one of ordinary skill in the art.

### Brief Description of Drawings

The foregoing and other aspects, features, and advantages of embodiments in the disclosure will become apparent from the following detailed description with reference to the accompanying drawings.
FIG. 1 is a diagram of a centrifugation device according to an embodiment.
FIG. 2 is a cross-sectional view of a centrifugation device according to an embodiment.
FIG. 3 is a perspective view of a piston for centrifugation according to an embodiment.
FIG. 4 is an exploded side view of a piston for centrifugation according to an embodiment.
FIG. 5 is an exploded cross-sectional view of a piston for centrifugation according to an embodiment.
FIG. 6 is a cross-sectional view of a piston body according to an embodiment.
FIG. 7 is a cross-sectional view of a valve according to an embodiment.
FIG. 8A is a cross-sectional view of a first fixing part according to an embodiment.
FIG. 8B is a plan view of a first fixing part according to an embodiment.
FIG. 9 is a cross-sectional view of a first state of a piston for centrifugation, according to an embodiment.
FIG. 10 is a cross-sectional view of a second state of a piston for centrifugation, according to an embodiment.
FIG. 11 is a cross-sectional view of a third state of a piston for centrifugation, according to an embodiment.
FIG. 12 is a cross-sectional view of a piston for centrifugation and a plunger according to an embodiment.
FIGS. 13 to 15 are diagrams illustrating example operations of a centrifugation device, according to an embodiment.

### Mode for the Invention

Hereinafter, embodiments will be described in detail with reference to the accompanying drawings. However, various alterations and modifications may be made to the embodiments. Here, the embodiments are not construed as limited to the disclosure. The embodiments should be understood to include all changes, equivalents, and replacements within the idea and the technical scope of the disclosure.

The terminology used herein is for the purpose of describing particular embodiments only and is not to be limiting of the embodiments. The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like constituent elements and a repeated description related thereto will be omitted. In the description of embodiments, detailed description of well-known related structures or functions will be omitted when it is deemed that such description will cause ambiguous interpretation of the present disclosure.

Also, in the description of the components, terms such as first, second, A, B, (a), (b) or the like may be used herein when describing components of the present disclosure. These terms are used only for the purpose of discriminating one constituent element from another constituent element, and the nature, the sequences, or the orders of the constituent elements are not limited by the terms. When one component is described as being "connected", "coupled", or "attached" to another component, it should be understood that one component may be connected or attached directly to another component, and an intervening component may also be "connected", "coupled", or "attached" to the components.

The same name may be used to describe an element included in the embodiments described above and an element having a common function. Unless otherwise mentioned, the descriptions on the embodiments may be applicable to the following embodiments and thus, duplicated descriptions will be omitted for conciseness.

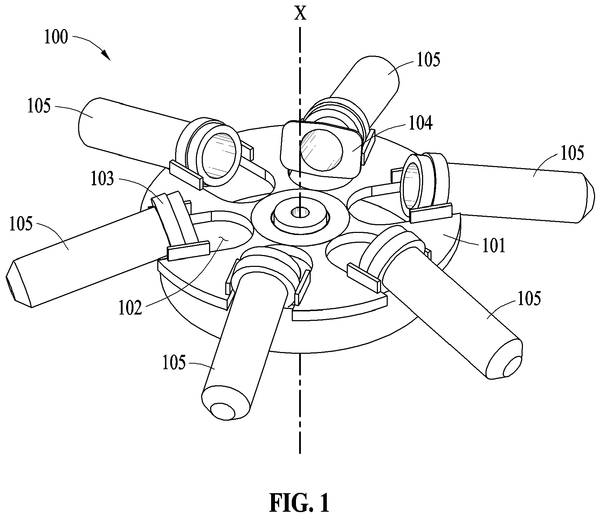

FIG. 1 is a diagram of a centrifugation device according to an embodiment.

Referring to FIG. 1, in a centrifugation environment, a centrifugation device 100 may remove impurities (e.g., oil, blood, medicine, and other impurities) from a biological tissue (e.g., fatty tissue) obtained from a living body (e.g., a human) and may obtain pure fatty tissue. The centrifugation device 100 may include a base 101 that is substantially flat, a plurality of cavities 102 arranged in a circumferential direction around the base 101, a plurality of hinges 103 rotatably connected to the base 101, a plurality of holders 105 respectively connected to the plurality of hinges 103 and arranged around a rotation shaft X, and at least one syringe 104 positioned in at least one holder 105 among the plurality of holders 105. The base 101 may include the rotation shaft X that is substantially located at the center of the base 101. Each of the plurality of holders 105 may be swung into and out of the corresponding cavity 102 by the corresponding hinge 103. The plurality of holders 105 may rotate around the rotation shaft X together with the base 101.

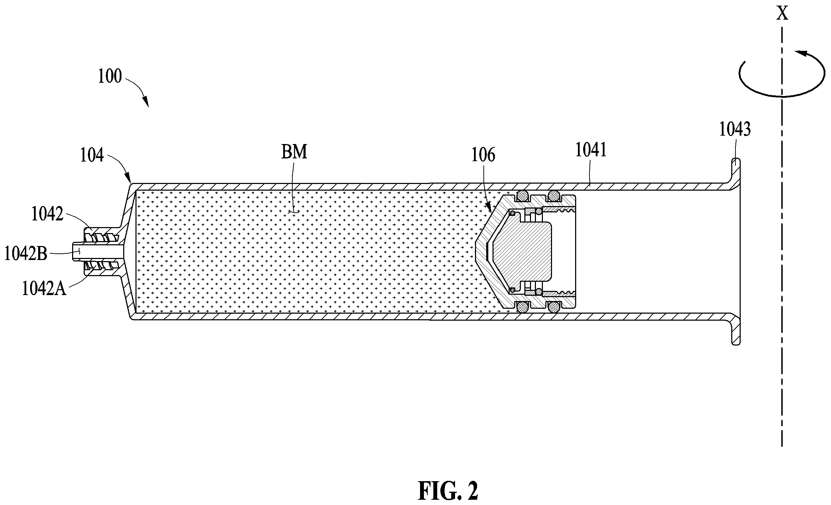

FIG. 2 is a cross-sectional view of a centrifugation device according to an embodiment.

Referring to FIG. 2, in the centrifugation environment, the centrifugation device 100 may include the syringe 104 configured to rotate with respect to the rotation shaft X. The syringe 104 may include a container 1041 having a cylindrical shape capable of holding a biological tissue (BM), a connector 1042 positioned at a first end part (e.g., a front end part of FIG. 2) of the container 1041 and configured to allow at least one first material (e.g., blood, medicine, water, and other materials with a relatively large proportion compared to pure fatty mass) to pass through and be removed, and a flange 1043 positioned at a second end part (e.g., a rear end part of FIG. 2) that is opposite to the first end part of the container 1041. The connector 1042 may include a fastening part 1042A configured to be coupled to an external removal device (not shown) and a discharge and injection passage 1042B through which a first material passes. The second end part of the container 1041, in which the flange 1043 is formed, may be open to the outside of the syringe 104. The BM obtained from the living body may be positioned inside the container 1041 through the discharge and injection passage 1042B, and a piston 106 may be positioned above (e.g., on the right side of FIG. 2) the BM. When the piston 106 is used, the piston 106 may press the BM, and at least one second material (e.g., oil and other materials with a relatively small proportion compared to pure fatty tissue) separated from the BM may escape toward the second end part of the container 1041 through the inside of the piston 106 in a state in which there is no centrifugal force.

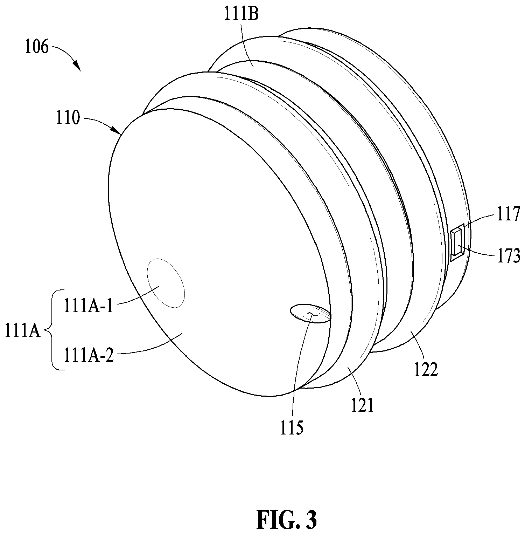

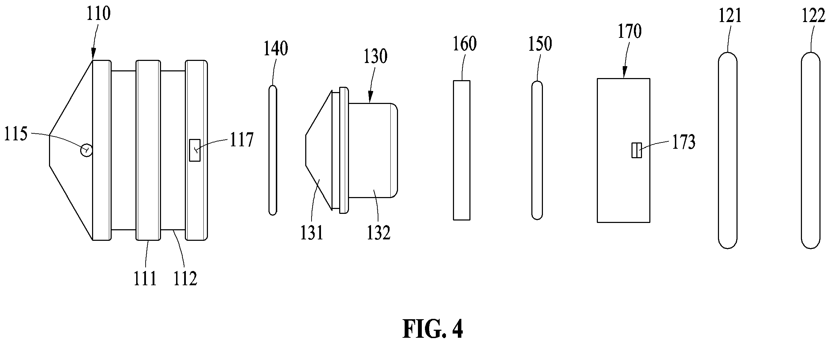

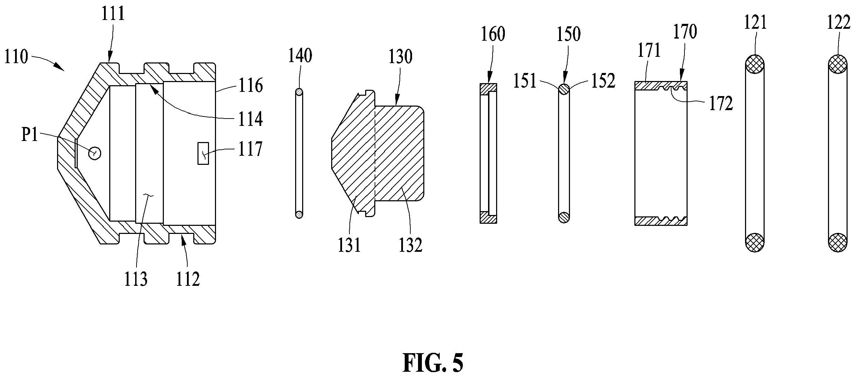

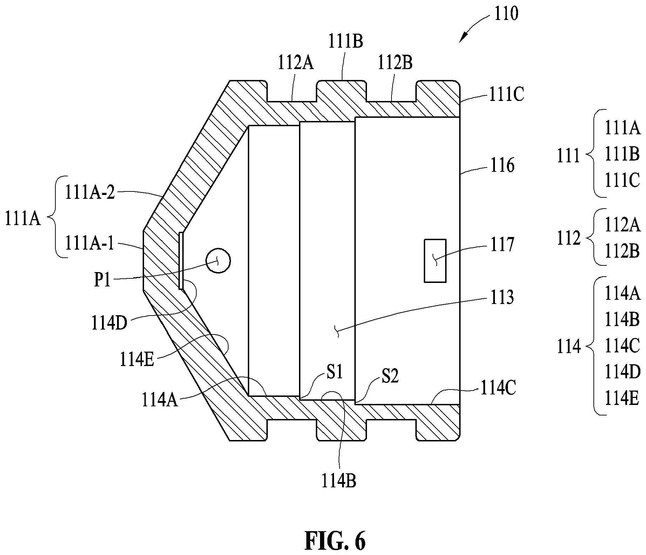

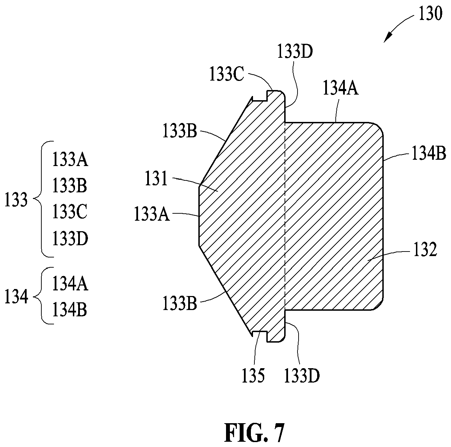

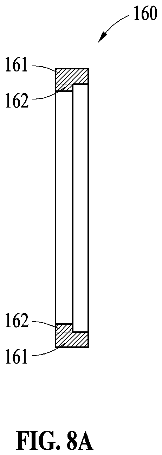

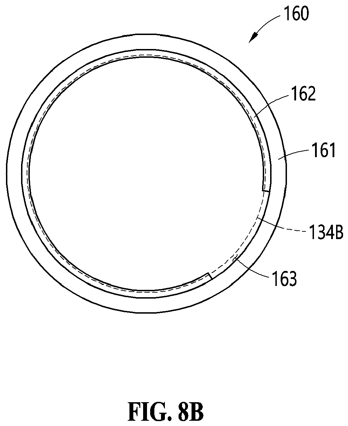

FIG. 2 is a cross-sectional view of a centrifugation device according to an embodiment. FIG. 3 is a perspective view of a piston for centrifugation according to an embodiment. FIG. 4 is an exploded side view of a piston for centrifugation according to an embodiment. FIG. 5 is an exploded cross-sectional view of a piston for centrifugation according to an embodiment. FIG. 6 is a cross-sectional view of a piston body according to an embodiment. FIG. 7 is a cross-sectional view of a valve according to an embodiment. FIG. 8A is a cross-sectional view of a first fixing part according to an embodiment. FIG. 8B is a plan view of a first fixing part according to an embodiment.

Referring to FIGS. 2 to 7 and FIGS. 8A and 8B, the piston 106 for centrifugation may include a piston body 110, a first outer sealing part 121, a second outer sealing part 122, a valve 130, a first inner sealing part 140, a second inner sealing part 150, a first fixing part 160, and a second fixing part 170.

The piston body 110 may be configured to move in the container 1041. For example, in the centrifugation environment, the piston body 110 may be configured to move in a direction away from the rotation shaft X and may press the BM obtained from the living body.

The piston body 110 may have an outer body surface 111 and an inner body surface 114 and may include a hollow part 113 defined by the inner body surface 114.

The outer body surface 111 may include a front body surface 111A that substantially forms the front of the piston body 110, a side body surface 111B that substantially forms the side of the piston body 110, and a rear body surface 111C that substantially forms at least a portion of the rear of the piston body 110. The front body surface 111A, the side body surface 111B, and the rear body surface 111C may be integrally and seamlessly formed.

The front body surface 111A may include a first front body surface 111A-1 and a second front body surface 111A-2 that is inclined to each of the first front body surface 111A-1 and the side body surface 111B. The first front body surface 111A-1, for example, when the piston 106 is positioned at a part (e.g., the front part, the left part of FIG. 2) of the container 1041 that is adjacent to the connector 1042, may press a space between the connector 1042 and the piston 106 and may facilitate the escape of materials (e.g., air, blood, medicine, and water) that may exist in the space through the discharge and injection passage 1042B. The first front body surface 111A-1 and the second front body surface 111A-2, for example, when the piston 106 is in the centrifugation environment, may press materials in front of the piston 106 and may guide the materials when the piston 106 moves to the front of the syringe 104. The first front body surface 111A-1 and the second front body surface 111A-2 may substantially form a truncated cone shape.

The piston body 110 may include a first opening 115 that allows materials on the front body surface 111A to flow into the hollow part 113. The first opening 115, for example, may be formed in the second front body surface 111A-2.

The piston body 110 may include a first passage P1 that fluidly connects the first opening 115 to the hollow part 113. The first passage P1, for example, may extend in a radial direction of the piston body 110 with a substantially cylindrical shape.

The side body surface 111B may face an inner wall of the container 1041. The piston body 110 may include an outer recess 112 formed in the side body surface 111B. For example, the outer recess 112 may include a first outer recess 112A formed in a first part of the side body surface 111B and a second outer recess 112B formed in a second part that is different from the first part of the side body surface 111B.

The rear body surface 111C may define a second opening 116 that is fluidly connected to each of the hollow part 113 and the outside of the piston body 110. The second opening 116 may allow materials in the hollow part 113 to flow out of the piston body 110. The size of the second opening 116 may be larger than that of the first opening 115.

The inner body surface 114 may include a first inner body surface 114A, a second inner body surface 114B, a third inner body surface 114C, a fourth inner body surface 114D, and a fifth inner body surface 114E.

The first inner body surface 114A, the second inner body surface 114B, and the third inner body surface 114C may be substantially parallel to the side body surface 111B. The first inner body surface 114A, the second inner body surface 114B, and the third inner body surface 114C may be positioned in order in a direction toward the second opening 116. The first inner body surface 114A, the second inner body surface 114B, the third inner body surface 114C, the fourth inner body surface 114D, and the fifth inner body surface 114E may be integrally and seamlessly formed.

A first step S1 may be formed between the first inner body surface 114A and the second inner body surface 114B. For example, the second inner body surface 114B may be formed to be recessed from the first inner body surface 114A. For example, the first inner body surface 114A may be formed to protrude from the second inner body surface 114B. In an embodiment, the second inner body surface 114B may be formed to protrude from the first inner body surface 114A. In an embodiment, the first inner body surface 114A and the second inner body surface 114B may be integrally and seamlessly formed substantially without the first step S1.

A second step S2 may be formed between the second inner body surface 114B and the third inner body surface 114C. For example, the third inner body surface 114C may be formed to be recessed from the second inner body surface 114B. For example, the second inner body surface 114B may be formed to protrude from the third inner body surface 114C. In an embodiment, the third inner body surface 114C may be formed to protrude from the second inner body surface 114B. In an embodiment, the second inner body surface 114B and the third inner body surface 114C may be integrally and seamlessly formed substantially without the second step S2.

The fourth inner body surface 114D may be substantially parallel to the second front body surface 111A-2. The fourth inner body surface 114E may be formed to be inclined with respect to the first inner body surface 114A and the fifth inner body surface 114D. The fifth inner body surface 114D may be substantially parallel to the first front body surface 111A-1. The fifth inner body surface 114D may be formed to be recessed with respect to the fourth inner body surface 114E.

The piston body 110 may include a first engaging part 117. The first engaging part 117 may at least partially engage with the second fixing part 170. The first engaging part 117 may be formed in the side body surface 111B. The first engaging part 117 may include a slot. In an embodiment, the first engaging part 117 may include a protrusion.

The first outer sealing part 121 and the second outer sealing part 122 may seal a gap between the piston body 110 and the container 1041. The first outer sealing part 121 and the second outer sealing part 122 may each have an annular shape. The first outer sealing part 121 may be positioned at the first outer recess 112A, and the second outer sealing part 122 may be positioned at the second outer recess 112B.

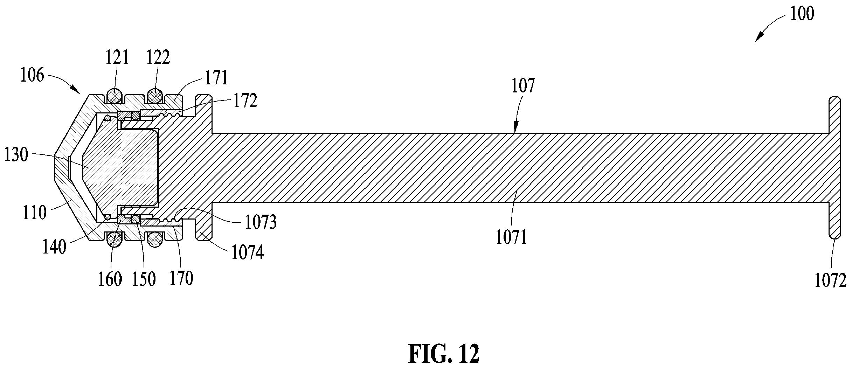

The valve 130 may allow or block the flow of at least one material between a front space of the piston body 110 and a rear space of the piston body 110. For example, in the centrifugation environment, the valve 130 may block the fluid passage formed between the front space of the piston body 110 and the rear space of the piston body 110 while moving with respect to the piston body 110 in a direction away from the rotation shaft X. For example, in an environment where the centrifugal force does not act on the valve 130 or an environment where the centrifugal force acting on the valve 130 is less than the sum of other forces (e.g., the gravity, frictional force, pressure on the front space of the piston 106 occurred by pushing the piston 106 without blocking the fluid passage, using a plunger 107 of FIG. 12, and/or pressure on the front space of the piston 106 occurred by pushing the piston 106 with the hand of a user without blocking the fluid passage, using the plunger 107 of FIG. 12), the valve 130 may open the fluid passage formed between the front space of the piston body 110 and the rear space of the piston body 110 while moving with respect to the piston body 110 toward the rear of the container 1041 facing the flange 1043. The weight of the valve 130 may be substantially the same as that of the piston body 110 or greater than that of the piston body 110.

The valve 130 may include a first valve body part 131 that substantially forms a front part of the valve 130 and a second valve body part 132 that substantially forms a rear part of the valve 130. The first valve body part 131 may include a first outer valve surface 133A, a second outer valve surface 133B, a third outer valve surface 133C, and a fourth outer valve surface 133D. The second valve body part 132 may include a fifth outer valve surface 134A and a sixth outer valve surface 134B. The first outer valve surface 133A, the second outer valve surface 133B, the third outer valve surface 133C, the fourth outer valve surface 133D, the fifth outer valve surface 134A, and the sixth outer valve surface 134B may form an outer valve surface 133 of the valve 130.

The first outer valve surface 133A may substantially form at least a portion of a front part of the first valve body part 131. The first outer valve surface 133A may substantially face the fourth inner body surface 114D. For example, the first outer valve surface 133A may be at least partially positioned at a recess in the fourth inner body surface 114D that is formed to be recessed to the fifth inner body surface 114E. The second outer valve surface 133B may substantially form at least a portion of the front part of the first valve body part 131. The second outer valve surface 133B may be formed to be inclined with respect to the first outer valve surface 133A and the third outer valve surface 133C. The second outer valve surface 133B may substantially face the fifth inner body surface 114E. The third outer valve surface 133C may substantially form a side part of the first valve body part 131. The third outer valve surface 133C may substantially face the first inner body surface 114A, the second inner body surface 114B, and/or the third inner body surface 114C. The fourth outer valve surface 133D may substantially form at least a portion of a rear part of the first valve body part 131. The fourth outer valve surface 133D may be formed between the third outer valve surface 133C and the fifth outer valve surface 134A.

The fifth outer valve surface 134A may substantially form a side part of the second valve body part 132. The fifth outer valve surface 134A may substantially face the first inner body surface 114A, the second inner body surface 114B, and/or the third inner body surface 114C. The sixth outer valve surface 134B may substantially form a rear part of the second valve body part 132.

The first outer valve surface 133A, the fourth outer valve surface 133D, and the sixth outer valve surface 134B may be substantially parallel to each other. The third outer valve surface 133C and the fifth outer valve surface 134A may be substantially parallel to each other.

The first outer valve surface 133A and the second outer valve surface 133B may be integrally and seamlessly formed. The second outer valve surface 133B and the third outer valve surface 133C may be formed discontinuously with respect to each other. The third outer valve surface 133C and the fourth outer valve surface 133D may be integrally and seamlessly formed. The fourth outer valve surface 133D, the fifth outer valve surface 134A, and the sixth outer valve surface 134B may be integrally and seamlessly formed. In an embodiment, the first outer valve surface 133A, the second outer valve surface 133B, the third outer valve surface 133C, the fourth outer valve surface 133D, the fifth outer valve surface 134A, and the sixth outer valve surface 134B may be integrally and seamlessly formed.

The valve 130 may include a recess part 135 formed in the first valve body part 131. The recess part 135, for example, may be formed between the second outer valve surface 133B and the third outer valve surface 133C.

The first inner sealing part 140 may be configured to seal a gap between the piston body 110 and the first valve body part 131. The first inner sealing part 140 may be at least partially positioned at the recess part 135. The first inner sealing part 140 may, for example, seal a gap between the fourth inner body surface 114E and the second outer valve surface 133B or a gap between the fourth inner body surface 114E and the third outer valve surface 133C. The first inner sealing part 140 may substantially have an annular shape.

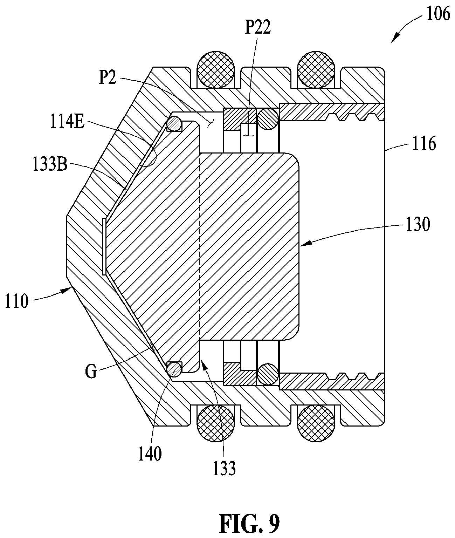

In the centrifugation environment, when the valve 130 receives the centrifugal force and moves with respect to the piston body 110 in a direction away from the rotation shaft X, and the first outer valve surface 133A and the second outer valve surface 133B substantially face the fourth inner body surface 114D and the fifth inner body surface 114E, respectively (see FIG. 9), the first inner sealing part 140 may be configured to seal a gap between the piston body 110 and the valve 130 while maintaining that a gap (e.g., a gap G of FIG. 9) is substantially present between the second outer valve surface 133B and the fifth inner body surface 114E by separating the second outer valve surface 133B and the fifth inner body surface 114E from each other. The first inner sealing part 140, for example, in a structure in which the first inner sealing part 140 is absent, may substantially prevent or reduce the leak that may occur because an inner body surface 114 (e.g., the fifth inner body surface 114E) of the piston body 110 and/or an outer valve surface (the second outer valve surface 133B) of the valve 130 do not substantially contact each other due to manufacturing tolerance.

The second inner sealing part 150 may be configured to seal a gap between the piston body 110 and a plunger (e.g., the plunger 107 of FIG. 12). The second inner sealing part 150 may contact at least a portion of an outer surface of the plunger (e.g., the plunger 107 of FIG. 12). The second inner sealing part 150 may be positioned on the second inner body surface 114B. The second inner sealing part 150 may substantially have an annular shape.

The first fixing part 160 may fix the second inner sealing part 150 to the second inner body surface 114B. The first fixing part 160 may be positioned on the second inner body surface 114B. In an example not shown, the first fixing part 160 may fix the second inner sealing part 150 to the second inner body surface 114B so that the second inner sealing part 150 is supported against the second step S2 in a structure in which the third inner body surface 114C is formed to protrude from the second inner body surface 114B.

The first fixing part 160 may restrict the movement of the valve 130. For example, in an environment where the centrifugal force does not act on the valve 130 or an environment where the centrifugal force acting on the valve 130 is less than the other forces (e.g., the gravity, frictional force, pressure on the front space of the piston 106 occurred by pushing the piston 106 without blocking the fluid passage, using the plunger 107 of FIG. 12, and/or pressure on the front space of the piston 106 occurred by pushing the piston 106 with the hand of the user without blocking the fluid passage, using the plunger 107 of FIG. 12), the valve 130 may move toward the second opening 116 with respect to the piston body 110, and the first fixing part 160 may prevent the valve 130 from being separated from the hollow part 113 so that the valve 130 is positioned in the hollow part 113.

The first fixing part 160 may include a first base part 161 and a protruding part 162 protruding from the first base part 161. The first base part 161 may be positioned on the second inner body surface 114B. The first base part 161 may be supported against the first step S1. The first base part 161 may support a first part 151 (e.g., the left part of FIG. 5) of the second inner sealing part 150. The protruding part 162 may be configured to contact a portion (e.g., the fourth outer valve surface 133D) of the first valve body part 131. The first base part 161 and the protruding part 162 may form a substantially annular structure of the first fixing part 160.

The protruding part 162 may protrude from an inner circumferential surface of the first base part 161. The protruding part 162 may be at least partially formed along the circumferential direction of the inner circumferential surface of the first base part 161. The protruding part 162 may form an inflow and outflow opening 163 while extending along the inner circumferential surface of the first base part 161. The inflow and outflow opening 163 may allow the inflow and outflow of materials through the inflow and outflow opening 163, for example, when one surface (e.g., the sixth outer valve surface 134B) of the valve 130 contacts the protruding part 162.

The second fixing part 170 may fix the second inner sealing part 150 to the second inner body surface 114B. The second fixing part 170 may be positioned on the third inner body surface 114C. The second fixing part 170 may fix the second inner sealing part 150 to the second inner body surface 114B so that the second inner sealing part 150 is supported by the first fixing part 160. In an example not shown, the second fixing part 170 may also fix the second inner sealing part 150 to the second inner body surface 114B so that the second inner sealing part 150 is supported by the first step S1. The second fixing part 170 may fix the second inner sealing part 150 in cooperation with the first fixing part 160.

The second fixing part 170 may include a second base part 171 positioned on the third inner body surface 114C. The second base part 171 may support a second part 152 (e.g., the right part of FIG. 5) of the second inner sealing part 150. The second base part 171 may form a substantially annular structure of the second fixing part 170.

The second fixing part 170 may include a first fastening part 172 configured to be fastened to an outer fastening means (e.g., the plunger 107 of FIG. 12). The first fastening part 172 may be at least partially formed in an inner surface of the second base part 171. The first fastening part 172 may include, for example, threads.

The second fixing part 170 may include a second engaging part 173. The second engaging part 173 may engage with at least a portion (e.g., the first engaging part 117) of the piston body 110. The second engaging part 173 may be at least partially formed in an outer surface of the second base part 171. The second engaging part 173 may include, for example, a protrusion.

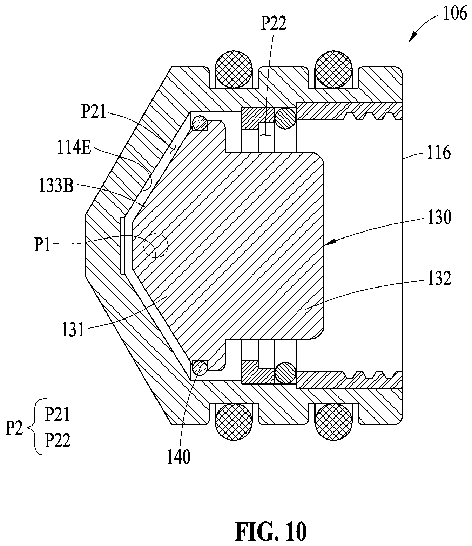

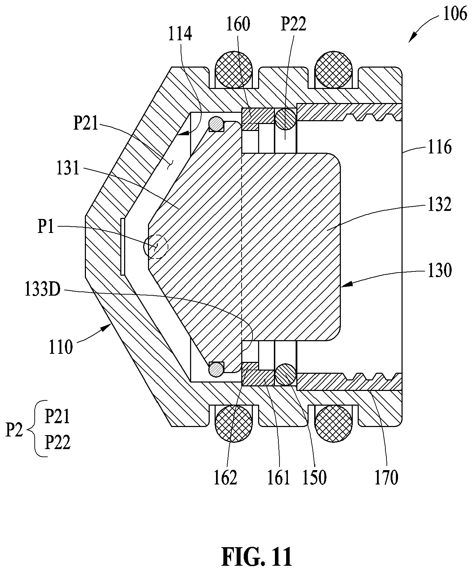

FIG. 9 is a cross-sectional view of a first state of a piston for centrifugation, according to an embodiment. FIG. 10 is a cross-sectional view of a second state of a piston for centrifugation, according to an embodiment. FIG. 11 is a cross-sectional view of a third state of a piston for centrifugation, according to an embodiment.

Referring to FIGS. 9 to 11, the piston 106 for centrifugation may be transferred from a first state (e.g., FIG. 9) that blocks communication between the first passage P1 and a second passage P2 to a second state (e.g., FIG. 10) that allows communication between the first passage P1 and the second passage P2. The piston 106 for centrifugation may be transferred from the first state (e.g., through the second state) to a third state (e.g., FIG. 11) that restricts the movement of the valve 130. The piston 106 for centrifugation may be transferred from the third state to the second state. The piston 106 for centrifugation may be transferred from the second state to the first state. The piston 106 for centrifugation may be transferred from the third state to the first state.

In the first state of the piston 106, in the centrifugation environment, the centrifugal force acting on the valve 130 with a first weight may be greater than the centrifugal force acting on the piston body 110 with a second weight that is less than the first weight. In the centrifugation environment, when the centrifugal force acting on the valve 130 is greater than the sum of other forces (e.g., the gravity, frictional force, pressure on the front space of the piston 106 that occurs by pushing the piston 106 without blocking the fluid passage, using the plunger 107 of FIG. 12, and/or pressure on the front space of the piston 106 that occurs by pushing the piston 106 with the hand of the user without blocking the fluid passage, using the plunger 107 of FIG. 12) acting on the valve 130, the valve 130 may move in a direction (e.g., to the front of the piston body 110) away from the second opening 116 with respect to the piston body 110. The first inner sealing part 140 installed in the valve 130 may contact the inner body surface 114 of the piston body 110 and may block the second passage P2 (e.g., a first sub-passage P21 between the fifth inner body surface 114E and the second outer valve surface 133B of FIG. 10) between the piston body 110 and the valve 130. The first inner sealing part 140 may separate at least a portion (e.g., the fifth inner body surface 114E) of the inner body surface 114 of the piston body 110 from at least a portion (e.g., the second outer valve surface 133B) of the outer valve surface 133 of the valve 130 and may form the gap G therebetween.

In the second state of the piston 106, in the centrifugation environment or an environment where the centrifugal force does not act, the valve 130 may move in a direction (e.g., to the rear of the piston body 110) toward the second opening 116 with respect to the piston body 110. The first inner sealing part 140 installed in the valve 130 may release the contact with the inner body surface 114 of the piston body 110 and may open the second passage P2 (e.g., the first sub-passage P21 between the fifth inner body surface 114E and the second outer valve surface 133B of FIG. 10) between the piston body 110 and the valve 130. At least one material in front of the piston body 110 may enter the first passage P1 through a first opening (e.g., the first opening 115 of FIG. 3), may flow through the first passage P1 and the second passage P2 (e.g., the first sub-passage P21 and a second sub-passage P22), and may escape to the rear of the piston body 110 through the second opening 116.

The piston 106 for centrifugation according to an embodiment may change a state between the first state (e.g., FIG. 9) that blocks the passages (P1, P2) and the third state (e.g., FIG. 11) that restricts the movement of the valve 130, or between the second state (e.g., FIG. 10) that opens the passages (P1, P2) and the third state (e.g., FIG. 11) that restricts the movement of the valve 130.

In the second state of the piston 106, in the centrifugation environment or an environment where the centrifugal force does not act, the valve 130 may move in a direction (e.g., to the rear of the piston body 110) toward the second opening 116 with respect to the piston body 110.

In the third state of the piston 106, the movement of the valve 130 may be restricted by the first fixing part 160 positioned on the inner body surface 114 (e.g., the second inner body surface 114B of FIG. 6) of the piston body 110. For example, at least a portion (e.g., the fourth outer valve surface 133D) of the first valve body part 131 among the first valve body part 131 and the second valve body part 132 may contact at least a portion of the first base part 161 or at least a portion of the protruding part 162. The first fixing part 160 may be supported by the second inner sealing part 150 and the second fixing part 170 respectively positioned on the inner body surface 114 (e.g., the second inner body surface 114B and the third inner body surface 114C) of the piston body 110.

In the third state of the piston 106, when the fourth outer valve surface 133D of the first valve body part 131 contacts the first base part 161, the movement of at least one material flowing through the first sub-passage P21 to the second sub-passage P22 may be suppressed or delayed.

FIG. 12 is a cross-sectional view of a piston for centrifugation and a plunger according to an embodiment.

Referring to FIGS. 2 to 12, the centrifugation device 100 may include the piston 106 for centrifugation including the piston body 110, the first outer sealing part 121, the second outer sealing part 122, the valve 130, the first inner sealing part 140, the second inner sealing part 150, the first fixing part 160, and the second fixing part 170, and the plunger 107. The second fixing part 170 may include the second base part 171 and the first fastening part 172.

The plunger 107 may be configured to couple to the piston 106. For example, the plunger 107 may press materials (e.g., blood, medicine, and water) positioned in front of the piston 106 in the container 1041 after coupling to the piston 106 and may allow the materials to escape from the container 1041 or enter the container 1041 through the discharge and injection passage 1042B of the connector 1042.

The plunger 107 may include a push rod 1071, a handle 1072 formed in a portion (e.g., the end part surface on the right side of FIG. 12) of the push rod 1071, a second fastening part 1073 formed in another portion (e.g., the outer surface on the left side of FIG. 12) of the push road 1071 and configured to be fastened to the first fastening part 172, and a seat 1074 extending in a radial direction of the push road 1071 and facing a portion (e.g., the rear body surface 111C) of the piston body 110. One of the first fastening part 1072 and the second fastening part 1073 may include male threads, and the other may include female threads.

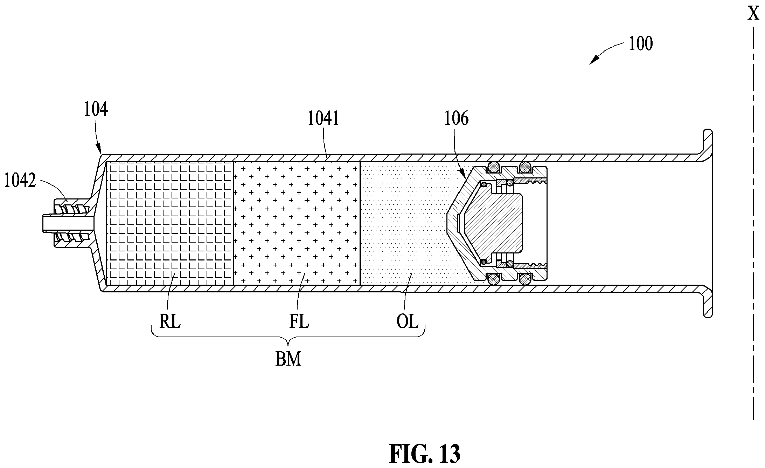

FIGS. 13 to 15 are diagrams illustrating example operations of a centrifugation device, according to an embodiment.

Referring to FIG. 2 and FIGS. 13 to 15, example operations of the centrifugation device 100 according to an embodiment are described.

Referring to FIG. 2, the BM obtained from the human body may be introduced through the front (e.g., the end part where the connector 1042 is positioned) of the container 1041, and the piston 106 may be positioned at the rear of the BM. Although not shown, the discharge and injection passage 1042B of the connector 1042 may be closed so that the BM may not escape to the outside of the container 1041. In the centrifugation environment, the syringe 104 and the piston 106 may rotate with respect to the rotation shaft X.

Referring to FIG. 13, during centrifugation, the BM in front of the piston 106 may be separated into a plurality of layers of materials forming the BM according to the proportion of the materials. For example, when viewed in a direction from the connector 1042 that is far away from the rotation shaft X to the piston 106, the BM may be sequentially separated into a first layer RL including at least one first material (e.g., blood, medicine, water) with a first weight, a second layer FL including at least one second material (e.g., fatty tissue from which a first material and a third material are removed) with a second weight that is less than the first weight, and a third layer OL including at least one third material (e.g., water, oil) with a third weight that is less than the second weight.

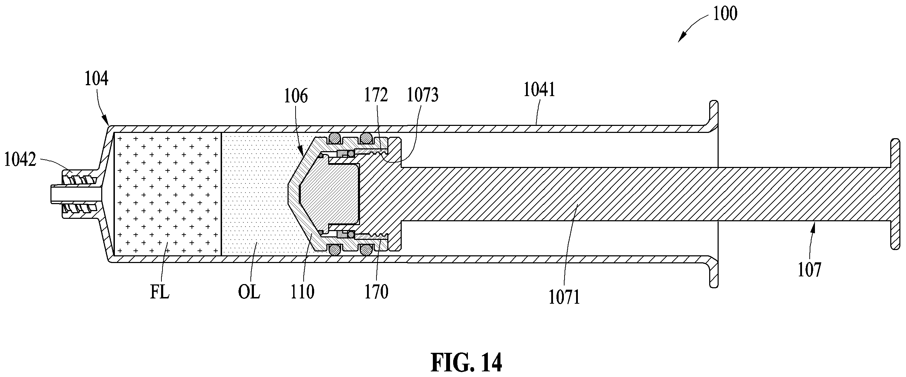

Referring to FIG. 14, the piston 106 and the plunger 107 may be coupled to each other. For example, the first fastening part 172 of the second fixing part 170 positioned on an inner body surface (e.g., the third inner body surface 114C of FIG. 6) of the piston body 110 and the second fastening part 1073 formed in the push rod 1071 of the plunger 107 may be fastened to each other. When the plunger 107 and the second fastening part 1073 are fastened to each other, and the piston 106 is pushed by the plunger 107, at least one material (e.g., the first material) in front of the piston 106 may be discharged to the outside of the container 1041 through the connector 1042, and the layer (e.g., the first layer RL of FIG. 13) of the material may be removed from the container 1041.

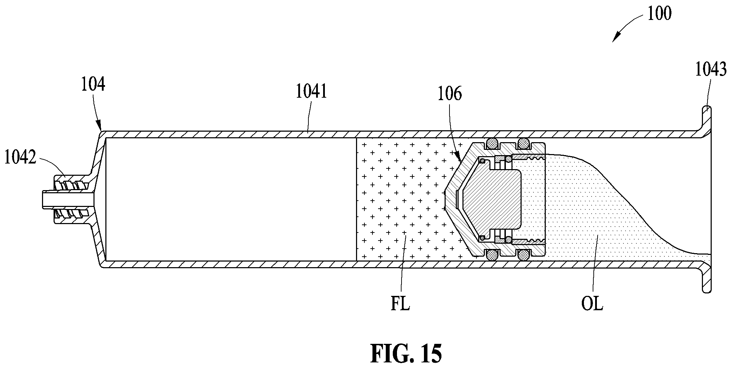

Referring to FIG. 15, the piston 106 and a plunger (e.g., the plunger 107 of FIG. 14) may be uncoupled from each other. In a state in which the plunger is uncoupled from the piston 106, the third layer OL of the third material (e.g., air, oil) positioned relatively close to the piston 106 may be discharged to the rear of the piston 106 in various methods. For example, in a state in which the second layer FL of the second material (e.g., fatty tissue or fatty lump from which the first material and the third material are removed) and the third layer OL of the third material exist in front of the piston 106, when the syringe 104 and the piston 106 are disposed in the centrifugation environment with relatively low centrifugal acceleration (e.g., approximately 200 g), the pressure in front of the piston 106 generated as the piston 106 moves by the centrifugal force becomes greater than the sealing force of the valve 130, and the third material of the third layer OL may escape to the rear of the piston 106 through the passages (e.g., the first passage P1 and the second passage P2 of FIG. 10) formed in the piston 106.

Moreover, in the second state (e.g., FIG. 10) and the third state (e.g., FIG. 11), the size of the gap between an inner body surface (e.g., the fifth inner body surface 114E) of a piston body and an outer valve surface (e.g., the second outer valve surface 133B) of a valve may be in a range of from about 5 micrometers (µm) to about 150 µm so that the size (e.g., the gap) of the flow space of the first sub-passage P21 between the inner body surface (e.g., the fifth inner body surface 114E) of the piston body (e.g., the piston body 110 of FIGS. 2 to 7 and FIGS. 8A and 8B) and the outer valve surface (e.g., the second outer valve surface 133B) of the valve (e.g., the valve 130 of FIGS. 2 to 7 and FIGS. 8A and 8B) is less than the size of the second material (e.g., fatty tissue) of the second layer FL in the front space of the piston 106. For example, a first fixing part (e.g., the first fixing part 160 of FIGS. 2 to 7 and FIGS. 8A and FIG. 8B) may restrict the movement of the valve (e.g., the valve 130 of FIGS. 2 to 7 and FIGS. 8A and 8B) to maintain the size range of the gap. When the first sub-passage P21 opens so that the flow space of the first sub-passage P21 is less than the size of the second material of the second layer FL, the second material of the second layer FL may not escape to the rear of the piston 106 through the passages (e.g., the first passage P1 and the second passage P2 of FIG. 10) formed in the piston 106 and may be positioned in front of the piston 106. In an embodiment, the size range of the gap may be substantially maintained by adjusting the mass of the valve 130 and/or the centrifugal force acting on the piston 106.

In another example, in a state in which an air pressure device is coupled to the rear (e.g., the flange 1043) of the container 1041 and the passages (e.g., the first passage P1 and the second passage P2 of FIG. 10) formed in the piston 106 open, the third material of the third layer OL in front of the piston 106 may be discharged to the rear of the piston 106 using the air pressure device.

In an embodiment, when the piston 106 is pushed to the front of the syringe 104 without coupling the plunger 107 and/or other control tools to the piston 106, the pressure in front of the piston 106 may increase, so the third material of the third layer OL in front of the piston 106 may be discharged to the rear of the piston 106. After the third material is removed and the piston 106 is removed from the syringe 104, the second material of the second layer FL may be obtained.

While this disclosure includes specific embodiments, it will be apparent to one of ordinary skill in the art that various changes in form and details may be made in these embodiments without departing from the spirit and scope of the claims and their equivalents. The embodiments described herein are to be considered in a descriptive sense only, and not for purposes of limitation. Descriptions of features or aspects in each embodiment are to be considered as being applicable to similar features or aspects in other embodiments. For example, suitable results may be achieved if the described techniques are performed in a different order, and/or if components in a described system, architecture, device, or circuit are combined in a different manner, and/or replaced or supplemented by other components or their equivalents.

Therefore, other implementations, other embodiments, and/or equivalents of the claims are within the scope of the following claims.

## Claims

1. A piston for centrifugation, the piston comprising:
a piston body having an outer body surface and an inner body surface, wherein the piston body comprises a hollow part defined by the inner body surface, wherein the piston body comprises a first opening positioned at the outer body surface, a second opening defined by at least a portion of the outer body surface and connected to the inner body surface and the hollow part, and a first passage connecting the first opening to the hollow part;
a valve configured to open or block the first passage and positioned at the hollow part to be spaced apart from the inner body surface, wherein the valve has an outer valve surface, wherein the outer valve surface defines the first passage and a second passage, wherein the first passage is positioned between the inner body surface and the outer valve surface, wherein the second passage is fluidly connected to the second opening, wherein the valve comprises a recess part formed in the outer valve surface; and
a first inner sealing part positioned at the recess part, in contact with the inner body surface, configured to space the outer valve surface apart from the inner body surface, and configured to, at a time of centrifugation, seal a gap between the inner body surface and the outer valve surface.

2. The piston of claim 1, further comprising:
a first fixing part positioned on the inner body surface and configured to restrict movement of the valve toward the second opening.

3. The piston of claim 2, wherein
the valve comprises:
a first valve body part comprising the recess part; and
a second valve body part connected to the first valve body part,
the first fixing part comprises:
a base part positioned on the inner body surface; and
a protruding part protruding from the base part and configured to contact the first valve body part.

4. The piston of claim 3, wherein the first valve body part contacts the protruding part so that a size of the gap between the inner body surface and the outer valve surface is from 5 micrometers (µm) to 150 µm.

5. The piston of claim 3, wherein
the second passage comprises:
a first sub-passage defined between the inner body surface and the first valve body part; and
a second sub-passage defined between the inner body surface and the second valve body part,
the valve contacts the first fixing part and is configured to suppress or delay movement of at least one material from the first sub-passage to the second sub-passage.

6. The piston of claim 2, wherein the inner body surface comprises a first inner body surface and a second inner body surface that is recessed from the first inner body surface and forms a first step with the first inner body surface, and the first fixing part is positioned on the second inner body surface.

7. The piston of claim 1, further comprising:
a second inner sealing part positioned on the inner body surface and comprising a first part and a second part that is opposite to the first part; and
a first fixing part positioned on the inner body surface, configured to support the first part of the second inner sealing part, and configured to fix the second inner sealing part to the inner body surface.

8. The piston of claim 7, further comprising:
a second fixing part positioned on the inner body surface, configured to support the second part of the second inner sealing part, and configured to fix the second inner sealing part to the inner body surface.

9. The piston of claim 8, wherein
the piston body comprises a first engaging part, and
the second fixing part comprises a second engaging part configured to engage with the first engaging part.

10. The piston of claim 8, wherein the second fixing part comprises a fastening part.

11. A centrifugation device with a rotation shaft, the centrifugation device comprising:
a syringe configured to rotate with respect to the rotation shaft, wherein the syringe comprises a container capable of holding a biological tissue; and
a piston for centrifugation movably positioned inside the container,
wherein the piston comprises:
a piston body having an outer body surface and an inner body surface, wherein the piston body comprises a hollow part defined by the inner body surface, wherein the piston body comprises a first opening positioned at the outer body surface, a second opening defined by at least a portion of the outer body surface and connected to the inner body surface and the hollow part, and a first passage connecting the first opening to the hollow part;
a valve configured to open or block the first passage and positioned at the hollow part to be spaced apart from the inner body surface, wherein the valve has an outer valve surface, wherein the outer valve surface defines the first passage and a second passage, wherein the first passage is positioned between the inner body surface and the outer valve surface, wherein the second passage is fluidly connected to the second opening, wherein the valve comprises a recess part formed in the outer valve surface; and
a first inner sealing part positioned at the recess part, in contact with the inner body surface, configured to space the outer valve surface apart from the inner body surface, and configured to, at a time of centrifugation, seal a gap between the inner body surface and the outer valve surface.

12. The centrifugation device of claim 11, wherein the piston further comprises:
a second inner sealing part positioned on the inner body surface and comprising a first part and a second part that is opposite to the first part;
a first fixing part positioned on the inner body surface, configured to support the first part of the second inner sealing part, and configured to fix the second inner sealing part to the inner body surface;
a second fixing part positioned on the inner body surface, configured to support the second part of the second inner sealing part, and configured to fix the second inner sealing part to the inner body surface; and
a plunger configured to be fastened to the second fixing part,
wherein the second inner sealing part contacts the plunger and is configured to seal a gap between the inner body surface and the plunger.
